(19) Europäisches Patentamt
European Patent Office
Office européen des brevets

(11) EP 0 816 344 B1

(12) EUROPÄISCHE PATENTSCHRIFT

(45) Veröffentlichungstag und Bekanntmachung des Hinweises auf die Patenterteilung:
**18.06.2003 Patentblatt 2003/25**

(51) Int Cl.[7]: **C07D 239/48**

(21) Anmeldenummer: **97114001.7**

(22) Anmeldetag: **25.04.1995**

(54) **Verfahren zur Herstellung von 2,5-Diamino-4,6-dichlorpyrimidin**

Process for the production of 2,5-diamino-4,6-dichloropyrimidine

Procédé de production de 2,5-diamino-4,6-dichloropyrimidine

(84) Benannte Vertragsstaaten:
**AT BE CH DE DK ES FR GB IE IT LI NL PT SE**

(30) Priorität: **27.04.1994 CH 129994**

(43) Veröffentlichungstag der Anmeldung:
**07.01.1998 Patentblatt 1998/02**

(62) Dokumentnummer(n) der früheren Anmeldung(en) nach Art. 76 EPÜ:
**95106220.7 / 0 684 236**

(73) Patentinhaber: **Lonza AG**
**3930 Visp (CH)**

(72) Erfinder:
- **Stucky, Gerhard, Dr.**
  **3902 Brig-Glis, (Kanton Wallis) (CH)**
- **Imwinkelried, René, Dr.**
  **3904 Naters, (Kanton Wallis) (CH)**

(74) Vertreter: **Winter, Brandl, Fürniss, Hübner, Röss, Kaiser, Polte Partnerschaft**
**Patent- und Rechtsanwaltskanzlei**
**Alois-Steinecker-Strasse 22**
**85354 Freising (DE)**

(56) Entgegenhaltungen:
- **C. TEMPLE, JR. ET AL.: "Preparation of 2,5-Diamino-4,6-dichlorpyrimidine" J. ORG. CHEM., Bd. 40, Nr. 21, Seiten 3141-3142, XP000566489**
- **CHEMICAL ABSTRACTS, vol. 89, no. 25, 18.Dezember 1978 Columbus, Ohio, US; abstract no. 215347s, C. TEMPLE, JR. ET AL.: "Preparation of 2,5-diamino-4,6-dichloropyrimidine via N-(4,6-dichloro-5-nitropyrimidin-2-yl)acet amide: The preparation of 2-aminopyrimidine intermediates" Seite 591; XP002044523 & NUCLEIC ACID CHEM., Nr. 1, Seiten 47-52,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 816 344 B1

## Beschreibung

Die Erfindung betrifft ein Verfahren zur Herstellung von 2,5-Diamino-4,6-dichlorpyrimidin der Formel

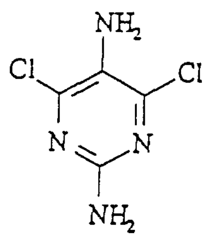

I

Diese Verbindung ist ein wertvolles Zwischenprodukt zur Herstellung von antiviralen Nukleotidderivaten.

Bekannt sind bisher N-5-geschützte 2,5-Diamino-4,6-dichlorpyrimidine, die als Zwischenprodukte zur Herstellung von antiviralen Nukleotidderivaten dienen (EP-A-0 552 758). Diese Verbindungen haben jedoch den Nachteil, daß sie sich schlecht zu den entsprechenden Nukleotidderivaten umsetzen lassen.

Im Unterschied zu diesen geschützten Pyrimidin-Derivaten kann das 2,5-Diamino-4,6-dichlorpyrimidin der Formel I auf einfache Weise und in guter Ausbeute in das entsprechende Nukleotidderivat überführt werden.

Aufgabe der Erfindung war es daher, ein wirtschaftliches Verfahren zur Herstellung von 2,5-Diamino-4,6-dichlorpyrimidin der Formel I bereitzustellen.

Diese Aufgabe wurde mit dem erfindungsgemäßen Verfahren nach Anspruch 1 gelöst.

In der ersten Verfahrensstufe wird ein Aminomalonester der allgemeinen Formel

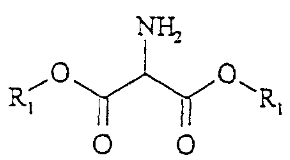

II

worin $R_1$ eine $C_1$-$C_6$-Alkylgruppe bedeutet, oder dessen Salz mit Guanidin oder dessen Salz in Gegenwart einer Base zum 2,5-Diamino-4,6-dihydroxypyrimidin der Formel

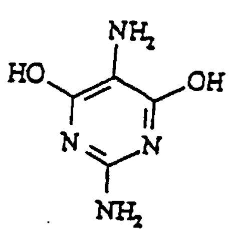

III

oder dessen Salz, cyclisiert.

Die als Edukte eingesetzten Aminomalonester der allgemeinen Formel II können auf bekannte Weise durch Aminierung der entsprechenden Malonester-Derivate erhalten werden.

Als Base wird zweckmäßig, entsprechend zur EP-A-0 552 758, ein Alkalimetallalkoholat wie beispielsweise Natrium- oder Kaliummethanolat oder Natrium- oder Kaliumethanolat verwendet. Vorzugsweise wird in situ gebildetes Natriummethanolat in Methanol oder Natriumethanolat in Ethanol angewendet.

Als Salze des Aminomalonesters und des Guanidins werden zweckmäßig deren Hydrochlorid- bzw. Hydrobromid-Salze verwendet.

Zweckmäßig erfolgt die Cyclisierung bei einer Temperatur zwischen Raumtemperatur und Rückflußtemperatur des entsprechenden Lösungsmittels, vorzugsweise bei Rückflußtemperatur.

Nach einer üblichen Umsetzungszeit zwischen 2 und 6 h kann dann gegebenenfalls das Zwischenprodukt gemäß Formel III durch übliche Aufbereitungsmethoden isoliert werden. Vorzugsweise erfolgt die Synthese des Endproduktes gemäß Formel I ohne Isolation des Zwischenproduktes gemäß Formel III.

Die zweite Verfahrensstufe erfolgt derart, daß man das Zwischenprodukt gemäß Formel III oder dessen Salz mit einem Chlorierungsmittel in Gegenwart eines Amids der allgemeinen Formel

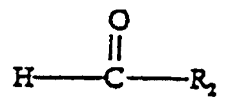

IV

zu einem 4,6-Dichlorpyrimidin der allgemeinen Formel

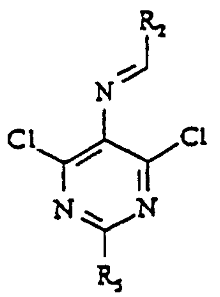

V

chloriert.

Der Substituent $R_5$ bedeutet $-NH_2$, -NH-CH=O oder $-N=CH-R_2$. Der Substituent $R_2$ bedeutet entweder

- einen 5- oder 6-gliedrigen N-Heterocycloalkylrest, der gegebenenfalls am Stickstoffatom substituiert ist, wie beispielsweise Piperidinyl-, Morpholinyl-, Thiomorpholinyl-, Pyrrolidinyl-, N-Methylpiperazinyl, vorzugsweise Piperidinyl- oder Pyrrolidinyl-, oder
- $NR_3R_4$, worin $R_3$ und $R_4$ gleich oder verschieden sind und eine $C_1$-$C_6$-Alkylgruppe wie beispielsweise Methyl-, Ethyl-, Propyl-, Isopropyl-, Butyl-, Pentyl- oder Hexyl-, vorzugsweise Methyl-, oder eine Benzylgruppe darstellen.

Der N-Heterocycloalkylrest kann zusätzlich andere Heteroatome, wie z.B. ein Sauerstoffatom, enthalten.

Demzufolge können als Amide der Formel IV N,N-Dimethylformamid, N,N-Diethylformamid, N,N-Diisopropylformamid, N-Formylpiperidin, N-Formylmorpholin, N-Formylthiomorpholin, N,N-Methylformylpiperazin oder N,N-Dibenzylformamid, vorzugsweise N,N-Dimethylformamid, N-Formylpiperidin oder N,N-Dibenzylformamid, angewendet werden.

Als Salze des Zwischenproduktes gemäß Formel III werden zweckmäßig dessen Hydrochlorid- bzw. Hydrobromid-Salze oder dessen Alkalimetallsalze wie beispielsweise dessen Natrium- oder Kaliumsalze verwendet.

Als Chlorierungsmittel können fachmännisch übliche angewendet werden, wie beispielsweise Phosphoroxychlorid, Thionylchlorid, Sulfurylchlorid, Phosphortrichlorid, Phosphorpentachlorid, Phosgen oder Diphosgen. Vorzugsweise wird als Chlorierungsmittel Phosphoroxychlorid verwendet.

Zweckmäßig werden das Chlorierungsmittel und das Amid (IV) im Molverhältnis 1 zu 0,55 bis 1 zu 10, vorzugsweise im Molverhältnis 1 zu 0,55 bis 1 zu 1, angewendet.

Die Chlorierung wird zweckmäßig bei einer Temperatur von 50°C bis Rückflußtemperatur des entsprechenden Lösungsmittels durchgeführt.

Als Lösungsmittel für die Chlorierung kann das zuvor beschriebene Amid angewendet werden. Die Chlorierung kann jedoch auch noch zusätzlich in einem inerten Lösungsmittel durchgeführt werden. Als inerte Lösungsmittel können beispielsweise Toluol, Xylol, Chloroform, Dichlormethan, Dichlorethan oder Chlorbenzol angewendet werden, vorzugsweise Toluol oder Dichlorethan.

Nach einer üblichen Umsetzungszeit von 3 bis 24 h kann das entsprechende 4,6-Dichlorpyrimidin der allgemeinen Formel V ($R_5 = -NH_2$) auf fachmännische Weise isoliert werden. Diese 4,6-Dichlorpyrimidine sind neue Zwischenprodukte zur Herstellung von 2,5-Diamino-4,6-dichlorpyrimidin. Bevorzugte Vertreter der 4,6-Dichlorpyrimidine V ($R_5 = -NH_2$) sind 4,6-Dichlor-N'-(dimethylaminomethylen)pyrimidin-2,5-diamin und 4,6-Dichlor-N'-(piperidin-1-ylmethylen)pyrimidin -2,5-diamin.

Je nach Wahl der Reaktions- oder Aufarbeitungsbedingungen können auch Vorstufen dieser 4,6-Dichlorpyrimidine (V) isoliert werden. Diese Vorstufen sind ebenfalls durch die allgemeine Formel V definiert. $R_5$ bedeutet dann

-NH-CH=O oder -N=CH-$R_2$, worin $R_2$ die genannte Bedeutung hat. Diese Vorstufen sind ebenfalls neue Zwischenprodukte zur Herstellung von 2,5-Diamino-4,6-dichlorpyrimidin. Bevorzugte Vertreter sind 4,6-Dichlor-N,N'-bis(dimethylaminomethylen)pyrimidin-2,5-diamin, 4,6-Dichlor-N,N'-bis(piperidin-1-ylmethylen)-pyrimidin-2,5-diamin bzw. N-[4,6-Dichlor-5-(dimethylaminomethylenamino)pyrimidin-2-yl]formamid.

In der dritten Verfahrensstufe werden die 4,6-Dichlorpyrimidine der allgemeinen Formel V mit einer Carbonsäure der allgemeinen Formel

$$R_6—COOH \qquad VI$$

worin $R_6$ eine $C_1$-$C_6$-Alkylgruppe, verzweigt oder unverzweigt, oder eine $C_3$-$C_6$-Cycloalkylgruppe bedeutet, in einer wäßrigen alkoholischen Lösung direkt zum Endprodukt gemäß Formel I umgesetzt.

Als Carbonsäure kann Essigsäure, Propionsäure, Buttersäure, Pentancarbonsäure, Hexancarbonsäure, Isobuttersäure, Pivalinsäure, Cyclopropancarbonsäure, Cyclopentancarbonsäure oder Cyclohexancarbonsäure angewendet werden. Zweckmäßig wird Essigsäure, Propionsäure oder Pivalinsäure angewendet.

Zweckmäßig wird die Carbonsäure in einer Konzentration von 20 bis 70 Vol.%, vorzugsweise von 25 bis 50 Vol.%, angewendet.

Als wäßrig alkoholische Lösung kann eine wäßrige Lösung aus Methanol, Ethanol, Propanol, Butanol, Pentanol oder Hexanol angewendet werden.

Zweckmäßig wird die Umsetzung in der dritten Stufe bei einer Temperatur von 50 bis 100°C, vorzugsweise bei einer Temperatur von 70 bis 90°C, durchgeführt.

Nach einer üblichen Umsetzungszeit von 1 bis 10 h kann dann das Endprodukt gemäß Formel I durch fachmännisch übliche Aufarbeitungsmethoden isoliert werden. Das Endprodukt gemäß Formel I kann im Unterschied zu den bekannten N-5-geschützten 2,5-Diamino-4,6-dichlorpyrimidinen (EP-A-0 552 758) auf einfache Weise und in guter Ausbeute in das entsprechende Nukleotidderivat überführt werden.

## Beispiele

### Beispiel 1

**Herstellung von 4,6-Dichlor-N'-(dimethylaminomethylen)-pyrimidin-2,5-diamin**

Eintopfverfahren:

Eine Suspension von 25 g (117 mmol) Aminomalonester-hydrochlorid in 50 ml Methanol wurde auf 10°C abgekühlt und mit 21,07 g Natriummethanolat (30%ig in Methanol) versetzt.

Diese Suspension wurde zu. einem Gemisch von 63,2 g (351 mmol) Natriummethanolat (30%ige Lösung in Methanol) und 12,55 g (128,7 mmol) Guanidin-hydrochlorid in 50 ml Methanol zugetropft. Das Reaktionsgemisch wurde zum Rückfluß erhitzt und anschließend für 16 Stunden bei dieser Temperatur gerührt. Zur warmen Suspension wurden anschließend 13,5 g (370 mmol) HCl-Gas eingeleitet. Anschließend wurde das Methanol abdestilliert. Während der Destillation wurden langsam insgesamt 200 ml Toluol zugetropft. Nachdem alles Methanol herausdestilliert war, wurden 71,6 g (468 mmol) $POCl_3$ und anschließend bei 80°C 34,2 g (468 mmol) Dimethylformamid zugetropft. Man ließ 17,5 Stunden bei 80°C rühren, kühlte dann auf Raumtemperatur ab und versetzte langsam mit 64,7 g $K_2CO_3$, gelöst in 150 ml Wasser. Man erwärmte erneut für 5 Stunden auf 50°C. Danach wurde mit 30%iger NaOH-Lösung der pH auf 7 gestellt, das Reaktionsgemisch gekühlt und das Produkt filtriert. Nach Waschen mit Wasser und Trocknen am Vakuum erhielt man 23,2 g (85%) reines Produkt als hellbraunen Feststoff.

| | |
|---|---|
| $^1$H-NMR (DMSO, 400 MHz) δ | 2,9-3,0 (2s, 6H);<br>6,9 (s, 2H);<br>7,6 (s, 1H). |
| $^{13}$C-NMR (DMSO, 100 MHz) | 33,5;<br>39,3;<br>130,3;<br>153,5;<br>157,0; |

(fortgesetzt)

| | |
|---|---|
| | 157,1. |

m.p.: 195°C (dec.).

**Beispiel 2**

**Herstellung von 4,6-Dichlor-N'-(dimethylaminomethylen)-pyrimidin-2,5-diamin**

**2.1 Herstellung von 4,6-Dichlor-N,N'-bis(dimethylaminomethylen)pyrimidin-2,5-diamin**

Eine Suspension von 4,46 g (25 mmol) Diaminodihydroxypyrimidin-hydrochlorid in 45 ml Toluol und 15,33 g (100 mmol) Phosphoroxychlorid wurde auf 90°C erwärmt. Innerhalb von 45 Minuten wurden 7,31 g (100 mmol) Dimethylformamid zugetropft. Anschließend wurde 20 Stunden bei 90°C gerührt. Man ließ abkühlen und versetzte das Reaktionsgemisch langsam mit 100 g 10%iger $K_2CO_3$-Lösung. Anschließend wurden 19,5 g festes $K_2CO_3$ zugegeben, damit der pH auf 7 anstieg. Das Produkt wurde mit drei Portionen Essigester extrahiert. Die gemeinsamen organischen Phasen wurden über $MgSO_4$ getrocknet und am Rotationsverdampfer eingeengt. Man erhielt 6,44 g eines hellbraunen Feststoffs. Ausbeute: 89%.

| | |
|---|---|
| $^{1}$H-NMR (DMSO, 400 MHz) δ | 2,9-3,1 (4s, 12 H);<br>7,6 (s, 1H);<br>8,5 (s, 1H). |
| $^{13}$C-NMR (DMSO, 100 MHz) | 33,5;<br>39,4;<br>40,4;<br>134,2;<br>153,0;<br>156,7;<br>158,0;<br>159,1. |

m.p.: 121,5 - 123°C.
CHN: ber. für $C_{10}H_{14}Cl_2N_6$: C 41,54, H 4,88, N 29,06; gef.: C 41,4, H 4,58, N 28,6.

**2.2 Herstellung von N-[4,6-Dichlor-5-(dimethylaminomethylenamino)pyrimidin-2-yl]formamid**

Eine Suspension von 3 g (10 mmol) des Produktes aus 2.1 in 10 ml 50%iger wäßriger Essigsäure wurde 4,5 Stunden bei Raumtemperatur gerührt. Anschließend wurde das Produkt filtriert und 2x mit je 10 ml Wasser gewaschen. Nach Trocknung am Vakuum erhielt man 2,18 g (83%) reines Produkt als weißen Feststoff.

| | |
|---|---|
| $^{1}$H-NMR (DMSO, 400 MHz) δ | 2,9-3,1 (2s, 6H);<br>7,7 (s, 1H);<br>9,2 (d, 1H);<br>11,2 (d, 1H). |
| $^{13}$C-NMR (DMSO, 100 MHz) | 33,6;<br>39,6;<br>136,9;<br>149,6;<br>153,4;<br>156,9;<br>162,5. |

m.p. : 172,5 - 174°C.
CHN: ber. für $C_8H_9Cl_2N_5O$: C 36,66, H 3,46, N 26,72; gef.: C 36,7, H 3,07, N 25,9.

**2.3 Herstellung von 4,6-Dichlor-N'-(dimethylaminomethylen)pyrimidin-2,5-diamin**

Eine Lösung von 1,85 g (7,1 mmol) des Produktes aus 2.2 in 25 ml 10%iger Salzsäure wurde auf 40°C erwärmt und bei dieser Temperatur 1,5 Stunden gerührt. Das Reaktionsgemisch wurde abgekühlt und mit 2M $K_2CO_3$ der pH auf 8,7 gestellt. Das ausgefallene Produkt wurde filtriert und mit Wasser gewaschen. Nach Trocknung am Vakuum erhielt man 1,52 g (91%) reines Produkt als weißen Feststoff.

**2.4 Herstellung von 4,6-Dichlor-N'-(dimethylaminomethylen)pyrimidin-2,5-diamin**

Eintopfverfahren:

Eine Suspension von 4,46 g (25 mmol) Diaminodihydroxypyrimidin-hydrochlorid in 90 ml Toluol und 15,33 g (100 mmol) Phosphoroxychlorid wurde auf 80°C erwärmt. Innerhalb von 60 Minuten wurden 7,31 g (100 mmol) Dimethylformamid zugetropft. Anschließend wurde 16 Stunden bei 80°C gerührt. Man ließ abkühlen und versetzte das Reaktionsgemisch mit 100 ml Wasser. Mit insgesamt 8,4 g $Na_2CO_3$ wurde der pH auf 10 gestellt. Man erwärmte auf 40°C und rührte das Reaktionsgemisch für 4 Stunden bei dieser Temperatur. Anschließend wurde auf Raumtemperatur abgekühlt, mit 30%iger NaOH-Lösung neutralisiert und das Produkt filtriert. Nach Waschen mit Wasser und Trocknen am Vakuum erhielt man 5,5 g (95%iges) Produkt als beigen Feststoff. Dies entspricht einer Ausbeute von 89%.

Beispiel 3

**Herstellung von 4,6-Dichlor-N'-(piperidin-1-ylmethylen)pyrimidin-2,5-diamin**

**3.1 Herstellung von 4,6-Dichlor-N,N'-bis(piperidin-1-ylmethylen)pyrimidin-2,5-diamin**

Eine Suspension von 3,57 g (20 mmol) Diaminodihydroxypyrimidin-hydrochlorid in 70 ml Toluol und 12,27 g (80 mmol) Phosphoroxychlorid wurde auf 80°C erwärmt. Innerhalb von 60 Minuten wurden 9,05 g (80 mmol) 1-Formylpiperidin zugetropft. Anschließend wurde 22 Stunden bei 80°C gerührt. Man ließ abkühlen und versetzte das Reaktionsgemisch mit 100 ml 1M $K_2CO_3$-Lösung. Anschließend wurde mit NaOH der pH auf 7 eingestellt. Das Produkt wurde mit drei Portionen Essigester extrahiert. Die gemeinsamen organischen Phasen wurden über $MgSO_4$ getrocknet und am Rotationsverdampfer eingeengt. Man erhielt 10,87 g eines Öls, welches noch sehr viel N-Formylpiperidin enthielt. Das Produkt konnte durch Aufschlämmen in Hexan und anschließende Filtration gereinigt werden. Ausbeute: >90%.

| | |
|---|---|
| $^1$H-NMR (DMSO, 300 MHz) δ | 8,5 (s, 1H);<br>7,7 (s, 1H);<br>3,4-3,8 (m, 8H);<br>1,5-1,9 (m, 12H). |

**3.2 Herstellung von 4,6-Dichlor-N'-(piperidin-1-ylmethylen)pyrimidin-2,5-diamin**

Eine Lösung von 9,9 g (18,2 mmol) des Produktes aus 3.1 in 73 g 10%iger HCl wurde zuerst 4,5 Stunden bei Raumtemperatur und anschließend 2 Stunden bei 47°C gerührt. Man kühlte ab und stellte den pH mit 30%iger NaOH auf 7 ein. Das Produkt wurde filtriert, mit Wasser gewaschen und am Vakuum getrocknet. Man erhielt 4,68 g (88%) Produkt als hellbraunen Feststoff.

| | |
|---|---|
| $^1$H-NMR (DMSO, 300 MHz) δ | 7,55 (s, 1H);<br>7,4 (s, 2H);<br>3,2-3,7 (m, 4H);<br>1,5-1,8 (m, 6H). |

**Beispiel 4**

**Herstellung von 2,5-Diamino-4,6-dichlorpyrimidin**

Ein Gemisch von 2,35 g (10 mmol) des Produktes aus Beispiel 1 in 5 g Pivalinsäure, 10 ml Methanol und 15 ml Wasser wurde 4,5 Stunden bei 80°C gerührt. Anschließend wurde der ausgefallene Feststoff filtriert und das Filtrat mit konzentrierter NaOH-Lösung neutralisiert. Es wurde mit Essigester extrahiert und die gemeinsamen organischen Phasen über $MgSO_4$ getrocknet. Nach Einengen am Rotationsverdampfer verblieben 1,40 g eines Produktegemisches, welches zu 65% (bestimmt nach $^1$H-NMR) aus dem gewünschten Produkt bestand (Ausbeute 51%). Das Produkt wurde nicht weiter gereinigt.

**Patentansprüche**

1. Verfahren zur Herstellung von 2,5-Diamino-4,6-dichlorpyrimidin der Formel

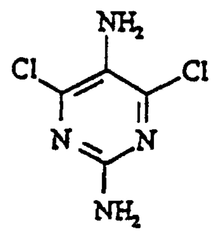

I

**dadurch gekennzeichnet, daß** man in der ersten Stufe einen Aminomalonester der allgemeinen Formel

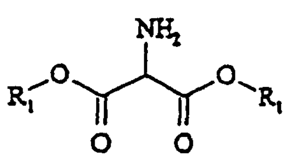

II

worin $R_1$ eine $C_1$-$C_6$-Alkylgruppe bedeutet, oder dessen Salz mit Guanidin oder dessen Salz in Gegenwart einer Base zum 2,5-Diamino-4,6-dihydroxypyrimidin der Formel

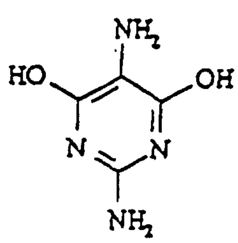

III

oder dessen Salz cyclisiert, dieses dann in der zweiten Stufe mit einem Chlorierungsmittel in Gegenwart eines Amids der allgemeinen Formel

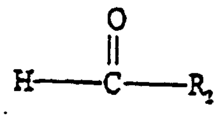

IV

worin $R_2$ einen 5- oder 6-gliedrigen N-Heterocycloalkylrest, der gegebenenfalls am Stickstoffatom substituiert ist, oder $-NR_3R_4$, worin $R_3$ und $R_4$ gleich oder verschieden sind und eine $C_1$-$C_6$-Alkylgruppe oder eine Benzylgruppe darstellen, bedeutet, zu einem 4,6-Dichlorpyrimidin der allgemeinen Formel

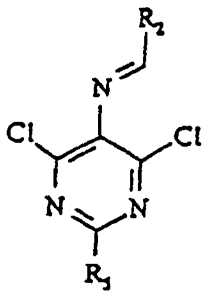

V

chloriert, worin $R_2$ die genannte Bedeutung hat und $R_5$ $-NH_2$, -NH-CH=O oder $-N=CH-R_2$, worin $R_2$ wie oben definiert ist, bedeutet, und dieses dann in der dritten Stufe mit einer Carbonsäure der allgemeinen Formel

$$R_6\text{—}COOH \qquad VI$$

worin $R_6$ eine $C_1$-$C_6$-Alkylgruppe, verzweigt oder unverzweigt, oder eine $C_3$-$C_6$-Cycloalkylgruppe bedeutet, in einer wäßrigen alkoholischen Lösung zum Endprodukt gemäß Formel I umsetzt.

2. Verfahren nach Anspruch 1, **dadurch gekennzeichnet, daß** man die Umsetzung ohne Isolation der Zwischenprodukte der Formel III durchführt.

3. Verfahren nach Anspruch 1 oder 2, **dadurch gekennzeichnet, daß** man in der ersten Stufe als Base ein Alkalimetallalkoholat verwendet.

4. Verfahren nach einem der Ansprüche 1 bis 3, **dadurch gekennzeichnet, daß** man in der zweiten Stufe als Chlorierungsmittel Phosphoroxychlorid verwendet.

5. Verfahren nach einem der Ansprüche 1 bis 4, **dadurch gekennzeichnet, daß** man in der zweiten Stufe als Amid Dimethylformamid, N-Formylpiperidin oder N,N-Dibenzylformamid verwendet.

6. Verfahren nach einem der Ansprüche 1 bis 5, **dadurch gekennzeichnet, daß** man die Chlorierung in der zweiten Stufe bei einer Temperatur von 50°C bis Rückflußtemperatur durchführt.

7. Verfahren nach einem der Ansprüche 1 bis 6, **dadurch gekennzeichnet, daß** man in der dritten Stufe als aliphatische Carbonsäure Essigsäure, Propionsäure oder Pivalinsäure verwendet.

8. Verfahren nach einem der Ansprüche 1 bis 7, **dadurch gekennzeichnet, daß** man die Umsetzung in der dritten Stufe bei einer Temperatur von 50 bis 100°C durchführt.

## Claims

1. A process for the production of 2,5-diamino-4,6-dichloropyrimidine of the formula

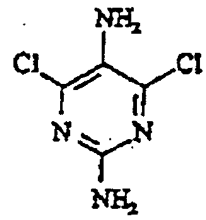

I

**characterised in that**, in the first stage, an aminomalonic ester of the general formula

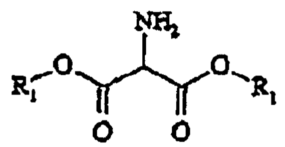

II

in which $R_1$ means a $C_1$-$C_6$ alkyl group, or the salt thereof is cyclised with guanidine or the salt thereof in the presence of a base to yield the 2,5-diamino-4,6-dihydroxypyrimidine of the formula

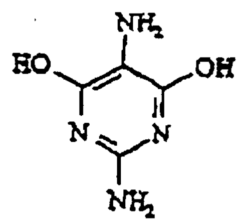

III

or the salt thereof, said latter compound is then chlorinated in the second stage with a chlorinating agent in the presence of an amide of the general formula

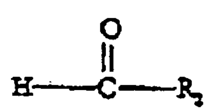

IV

in which $R_2$ is a 5- or 6-membered N-heterocycloalkyl residue, which is optionally substituted on the nitrogen atom, or $-NR_3R_4$, in which $R_3$ and $R_4$ are identical or different and represent a $C_1$-$C_6$ alkyl group or a benzyl group, to yield a 4,6-dichloropyrimidine of the general formula

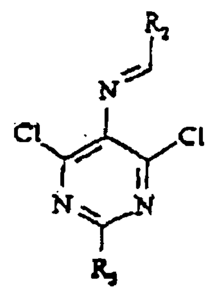

V

in which $R_2$ has the stated meaning and $R_5$ means $-NH_2$, -NH-CH=O or $-N=CH-R_2$, in which $R_2$ is defined as above, and said latter compound is then reacted in the third stage with a carboxylic acid of the general formula

$R_6$-COOH VI

in which $R_6$ means a $C_1$-$C_6$ alkyl group, branched or unbranched, or a $C_3$-$C_6$ cycloalkyl group, in an aqueous alcoholic solution to yield the final product according to the formula I.

**2.** A process according to claim 1, **characterised in that** the reaction is performed without isolating the intermediates of the formula III.

**3.** A process according to claim 1 or 2, **characterised in that** an alkali metal alkoxide is used as the base in the first stage.

**4.** A process according to one of claims 1 to 3, **characterised in that** phosphorus oxychloride is used as the chlorinating agent in the second stage.

**5.** A process according to one of claims 1 to 4, **characterised in that** dimethylformamide, N-formylpiperidine or N, N-dibenzylformamide is used as the amide in the second stage.

**6.** A process according to one of claims 1 to 5, **characterised in that** the chlorination in the second stage is performed at a temperature of 50°C to reflux temperature.

**7.** A process according to one of claims 1 to 6, **characterised in that** acetic acid, propionic acid or pivalic acid is used as the aliphatic carboxylic acid in the third stage.

**8.** A process according to one of claims 1 to 7, **characterised in that** the reaction in the third stage is performed at a temperature of 50 to 100°C.

## Revendications

**1.** Procédé de préparation de 2,5-diamino-4,6-dichloropyrimidine de formule :

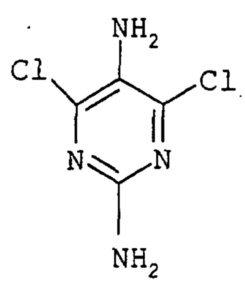

I

**caractérisé en ce que** l'on cyclise, dans la première étape, un ester aminomalonique de formule générale

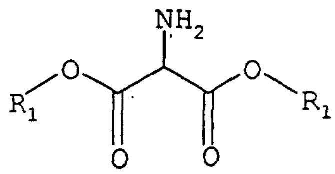

II

dans laquelle $R_1$ représente un groupe alkyle en $C_1$-$C_6$, ou son sel avec de la guanidine ou son sel en présence d'une base, en 2,5-diamino-4,6-dihydroxypyrimidine de formule

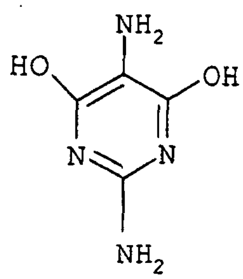

III

ou son sel, **en ce que** l'on chlore celui-ci dans la deuxième étape avec un agent de chloration en présence d'un amide de formule générale

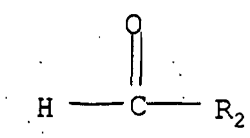

IV

dans laquelle $R_2$ représente un résidu N-hétérocycloalkyle à 5 ou 6 éléments, qui est éventuellement substitué sur l'atome d'azote, ou $-NR_3R_4$, où $R_3$ et $R_4$ sont identiques ou différents et représentent un groupe alkyle en $C_1$-$C_6$ ou un groupe benzyle, en 4,6-dichloropyrimidine de formule générale

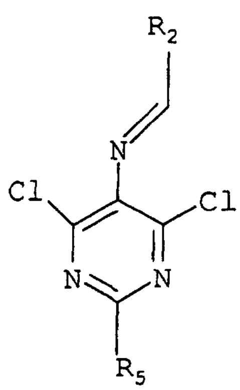

V

dans laquelle $R_2$ a la signification mentionnée et $R_5$ représente $-NH_2$, -NH-CH=O ou $-NH=CH-R_2$, où $R_2$ est tel que défini précédemment, et que l'on fait réagir celui-ci, dans la troisième étape, avec un acide carboxylique de

formule générale

$$R_6\text{—}COOH \qquad VI$$

où $R_6$ représente un groupe alkyle en $C_1$-$C_6$, ramifié ou non ramifié, ou un groupe cycloalkyle en $C_3$-$C_6$, dans une solution alcoolique aqueuse en produit final selon la formule 1.

**2.** Procédé selon la revendication 1, **caractérisé en ce que** l'on réalise la réaction sans isolation des produits intermédiaires de formule III.

**3.** Procédé selon la revendication 1 ou 2, **caractérisé en ce que** l'on utilise dans la première étape comme base un alcoolate de métal alcalin.

**4.** Procédé selon l'une quelconque des revendications 1 à 3, **caractérisé en ce que** l'on utilise dans la deuxième étape du phosphoroxychlorure comme agent de chloration.

**5.** Procédé selon l'une quelconque des revendications 1 à 4, **caractérisé en ce que** l'on utilise dans la deuxième étape comme amide le diméthylformamide, la N-formylpipéridine ou le N,N-dibenzylformamide.

**6.** Procédé selon l'une quelconque des revendications 1 à 5, **caractérisé en ce que** l'on réalise la chloration dans la deuxième étape à une température située entre 50°C et la température de reflux.

**7.** Procédé selon l'une quelconque des revendications 1 à 6, **caractérisé en ce que** l'on utilise dans la troisième étape comme acide carboxylique aliphatique l'acide acétique, l'acide proprionique ou l'acide de pivaline.

**8.** Procédé selon l'une quelconque des revendications 1 à 7, **caractérisé en ce que** l'on réalise la réaction dans la troisième étape à une température entre 50 et 100°C.